(19) Europäisches Patentamt
European Patent Office
Office européen des brevets

(11) EP 2 044 641 B1

(12) **EUROPEAN PATENT SPECIFICATION**

(45) Date of publication and mention
of the grant of the patent:
**25.12.2013 Bulletin 2013/52**

(51) Int Cl.:
*H01M 4/88* (2006.01)    *H01M 8/12* (2006.01)
*H01M 8/24* (2006.01)    *H01M 8/02* (2006.01)
*H01M 4/86* (2006.01)

(21) Application number: **07704961.7**

(22) Date of filing: **23.01.2007**

(86) International application number:
**PCT/GB2007/000182**

(87) International publication number:
**WO 2007/093759 (23.08.2007 Gazette 2007/34)**

(54) **A SOLID OXIDE FUEL CELL MODULE WITH MULTILAYERED ANODE**

FESTOXID-BRENNSTOFFZELLENMODUL MIT MEHRSCHICHTIGER ANODE

MODULE DE PILES A COMBUSTIBLE A OXYDE SOLIDE DOTE D'UNE ANODE MULTICOUCHE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **14.02.2006 GB 0602842**

(43) Date of publication of application:
**08.04.2009 Bulletin 2009/15**

(73) Proprietor: **LG Fuel Cell Systems, Inc.
North Canton OH 44720 (US)**

(72) Inventors:
• **DELAFORCE, Philip, Mark
Derby DE2 3DL (GB)**
• **HART, Nigel, Thomas
Singapore 309888 (SG)**
• **YEOMANS, Julie, Anne
Surrey GU15 1AG (GB)**
• **WRIGHT, Gary, John
Derby DE22 3AW (GB)**
• **JORGER, Michael, Bernhard
Derby DE24 8NN (GB)**

(74) Representative: **Vaughan, Christopher Tammo
Harrison Goddard Foote LLP
Belgrave Hall
Belgrave Street
Leeds LS2 8DD (GB)**

(56) References cited:
EP-A- 1 732 157      WO-A-01/24300
WO-A-97/35349        WO-A-2004/013925
JP-A- 7 245 107      US-A- 5 035 962
US-A- 5 273 837      US-A1- 2002 028 367

## Description

The present invention relates to a solid oxide fuel cell and a solid oxide fuel cell module.

JP07245107A discloses a solid oxide fuel cell comprising an air electrode, a fuel electrode and an electrolyte. The fuel electrode comprises a first layer arranged on the electrolyte, a second layer on the first layer and a third layer on the second layer. The first layer comprises a metal and ceramic cermet, the second layer comprises a porous metal and the third layer comprises a metal and ceramic cermet.

US5273837 discloses a solid oxide fuel cell comprising a flexible electrolyte, an anode electrode and a cathode electrode. The anode electrode comprises a single layer bonded to the electrolyte. The anode electrode comprises an anode grid which extends completely through the thickness of the anode electrode. The anode grid comprises a nickel mesh and this is permanently bonded to the electrolyte and the mesh acts as a current collector for the anode electrode. An electrode material for the anode electrode consisting of a mixture of nickel and zirconia is applied to fill the openings in the nickel mesh.

Solid oxide fuel cells comprising functionally graded anode electrodes and functionally graded cathode electrodes are known. The functionally graded anode and cathode electrodes generally comprise a first layer on the electrolyte and a second layer on the first layer. The first layer is arranged to optimise the electrochemical activity at the electrolyte and the second layer is arranged to provide electronic conduction perpendicular to the layers of the solid oxide fuel cells to allow current to flow from one solid oxide fuel cell to an adjacent solid oxide fuel cell via an interconnector. The second layers provide uniform current collection across the solid oxide fuel cells.

Solid oxide fuel cell modules comprising a plurality of solid oxide fuel cells connected in electrical series are known. The solid oxide fuel cells are connected in series by interconnectors.

The anode electrodes and cathode electrodes require coefficients of thermal expansion (CTE) close to that of the electrolyte as well as adequate adherence, or good interfacial bonding, with the electrolyte. Also, the anode electrodes and cathode electrodes are exposed to different operating environments and this gives rise to different material requirements for the anode electrodes and the cathode electrodes. The anode electrodes and cathode electrodes need to be thermally stable at high temperatures, chemically stable with respect to the electrolyte at high temperatures, have high electrical conductivity and be catalytic in either an oxidising or reducing atmosphere. Furthermore, the anode electrodes and cathode electrodes must be porous to allow for the flow of gaseous products and reactants to and from the reaction sites. All of these requirements limit the number of materials available for selection for use as anode electrodes and the cathode electrodes.

There are no materials available for selection, which satisfy all of the material requirements for use as anode electrodes and cathode electrodes and thus it is necessary to modify the materials. The generally accepted method of modifying the properties of a material is to mix the material with another material to produce a composite material. Thus, presently the anode electrodes and cathode electrodes of a solid oxide fuel cell comprise composite materials, also known as cermets, and these composite materials comprise two or more materials.

An anode electrode generally comprises a mixture of nickel and yttria stabilised zirconia (YSZ). The proportions of nickel and yttria stabilised zirconia (YSZ) is varied to adjust the coefficient of thermal expansion of the anode electrode to any value between that of nickel and yttria stabilised zirconia (YSZ). A sufficient proportion of nickel must be present in the anode electrode in order for the anode electrode to have good conductivity. In the end the proportions of nickel and yttria stabilised zirconia (YSZ) in the anode electrode is a compromise between the requirement for a coefficient of thermal expansion (CTE) matching that of the electrolyte, normally yttria stabilised zirconia, and the requirement for high electrical conductivity. In addition, because the anode electrode must be porous, the presence of pores in the anode electrode acts as an insulator and reduces the electrical conductivity of the anode electrode.

The cathode electrode has similar requirements to the anode electrode and the cathode electrode generally comprises strontium doped lanthanum manganite composite material or cermet.

The conductance of an object is expressed by equation (1), where G is the conductance of the object, ρ is the materials resistivity, L is the length of the object the current flows along and A is the cross-sectional area of the object. Resistivity, ρ, is the reciprocal of conductivity σ. Replacing conductivity for resistivity yields equation (2).

$$(1) \quad G = 1/\rho \; (A/L)$$

and

$$(2) \quad G = \sigma \; (A/L)$$

The solid oxide fuel cells described in our published International patent application WO0229917A are arranged on an inert porous substrate. In this arrangement the geometry of the current collecting layers connecting the adjacent solid oxide fuel cells together has a very small cross-sectional area to length ratio.

The ideal geometry to maximise conductance is to maximise the cross-sectional area to length ratio, by having a small length and large cross-sectional area.

In the solid oxide fuel cells described in our published International patent application WO0229917 the dimensions of the current collecting layer is restricted in terms of width and length, because these are directly related to the solid oxide fuel cell geometry and electrolyte size and printing tolerances. The thickness of the current collecting layer is not governed by this geometry, but the thickness must not be excessively large in magnitude because a thick layer affects the mechanical integrity.

As mentioned previously functionally graded anode and cathode electrodes are known. The conventional method to increase conductivity of the anode electrode and cathode electrode is to provide the second layers with enhanced electronic conduction. These second layers, known as current collector layers, must be porous to allow product and reactant gases to flow to and from the reaction sites.

Nickel is predominantly used in solid oxide fuel cells for the anode electrodes because nickel has good catalytic and electronic conductivity properties at high temperatures. Thus nickel would be a good material for the current collector layer.

However, nickel and nickel oxide have a coefficient of thermal expansion that is larger than yttria stabilised zirconia (YSZ) and nickel and nickel oxide undergo a significant volume change when oxidised and reduced respectively. Additionally, with time at high temperatures small nickel particles, in a current collector layer or anode layer, tend to coarsen due to the thermodynamic driving force to decrease the free energy by minimising the surface area, which alters the microstructure so that there is less percolation of the nickel particles. This leads to a decrease in electrical conductivity of the current collector layer or anode layer, with time. The rate of coarsening of the nickel particles is dependent upon the size of the nickel particles, smaller nickel particles coarsen at a faster rate than larger nickel particles.

The problem is there is no suitable material for the current collector layer for the anode electrode with suitable in plane conductance, thermal cycling stability and re-oxidation cycling stability.

Accordingly the present invention seeks to provide a novel solid oxide fuel cell, which reduces, preferably overcomes, the above-mentioned problem.

Accordingly the present invention provides a solid oxide fuel cell module comprising a plurality of solid oxide fuel cells, each fuel cell comprising an anode electrode, an electrolyte and a cathode electrode, a plurality of interconnectors being arranged to electrically connect the fuel cells in electrical series, each interconnector electrically connecting an anode electrode of one fuel cell to a cathode electrode of an adjacent fuel cell, the anode electrode comprising at least three layers, a first layer being arranged on the electrolyte, a second layer being arranged on the first layer and a third layer being arranged on the second layer, the first layer comprising a metal and ceramic cermet, the third layer comprising a metal and ceramic cermet, wherein the second layer comprising a current collector and a metal and ceramic cermet, and the current collector being embedded in the metal and ceramic cermet of the second layer, the current collector comprising a metal and ceramic cermet, the metal and ceramic cermet of the current collector having a higher concentration of metal than the concentration of metal in the metal and ceramic cermet of the first layer, second layer and third layer or the metal and ceramic cermet of the current collector having different sized metal and ceramic particles than the sizes of the metal and ceramic particles of the metal and ceramic cermet of the first layer, second layer and third layer or the metal and ceramic cermet of the current collector having less porosity than the metal and ceramic cermet of the first layer, second layer and third layer.

Preferably the metal of the cermet comprising at least one of copper, nickel, palladium, platinum, rhenium or rhodium.

Preferably the ceramic of the cermet comprising at least one of yttria stabilised zirconia, ceria or perovskite.

The current collector may comprise metal and ceramic cermet, the metal and ceramic cermet of the current collector having different shaped and/or sized metal and ceramic particles than the shapes and/or sizes of the metal and ceramic particles of the metal and ceramic cermet of the first layer, second layer and third layer.

The current collector may comprise metal and ceramic cermet, the metal and ceramic cermet of the current collector containing less pore formers than the metal and ceramic cermet of the first layer, second layer and third layer.

The first layer may comprise a nickel and ceramic cermet, the second layer comprising a current collector embedded in a nickel and ceramic cermet and the third layer comprising a nickel and ceramic cermet.

The current collector may comprise nickel and ceramic cermet, the nickel and ceramic cermet of the current collector having a higher concentration of nickel than the concentration of nickel in the nickel and ceramic cermet of the first layer, second layer and third layer.

The current collector may comprise nickel and ceramic cermet, the nickel and ceramic cermet of the current collector having different sized nickel and ceramic particles than the sizes of the nickel and ceramic particles of the nickel and ceramic cermet of the first layer, second layer and third layer.

The current collector may comprise nickel and ceramic cermet, the nickel and ceramic cermet of the current collector having less porosity than the nickel and ceramic cermet of the first layer, second layer and third layer.

The current collector may comprise nickel and ceramic cermet, the nickel and ceramic cermet of the current collector having different shaped and/or sized nickel and ceramic particles than the shapes and/or sizes of the nickel and ceramic particles of the nickel and ceramic cermet of the first layer, second layer and third layer.

The current collector may comprise nickel and ceramic cermet, the nickel and ceramic cermet of the current collector containing less pore formers than the nickel and ceramic cermet of the first layer, second layer and third layer.

Preferably the second layer comprises a current collector mesh embedded in a metal and ceramic cermet.

The second layer may comprise a hexagonal current collector mesh in a metal and ceramic cermet. The second layer may comprise a sinusoidal current collector mesh in a metal and ceramic cermet.

The second layer may comprise a plurality of discontinuous current collector members in a metal and ceramic cermet.

The nickel and ceramic cermet may comprise nickel and yttria stabilised zirconia cermet, nickel and ceria cermet or nickel and perovskite cermet.

The anode electrode may comprise a fourth layer arranged on the third layer and a fifth layer arranged on the fourth layer, the fourth layer comprising a current collector embedded in a metal and ceramic cermet and the fifth layer comprising a metal and ceramic cermet.

The anode electrode may comprise a sixth layer arranged on the fifth layer and a seventh layer arranged on the sixth layer, the sixth layer comprising a current collector embedded in a metal and ceramic cermet and the seventh layer comprising a metal and ceramic cermet.

The present invention will be more fully described by way of example with reference to the accompanying drawings in which:-

Figure 1 shows a solid oxide fuel cell stack according to the present invention.
Figure 2 shows an enlarged view of a solid oxide fuel cell shown in Figure 1.
Figure 3 shows a section along line A-A of the solid oxide fuel cell shown in Figure 2.
Figure 4 shows an alternative section along line A-A of the solid oxide fuel cell shown in Figure 2.
Figure 5 shows a further alternative section along line A-A of the solid oxide fuel cell shown in Figure 2.

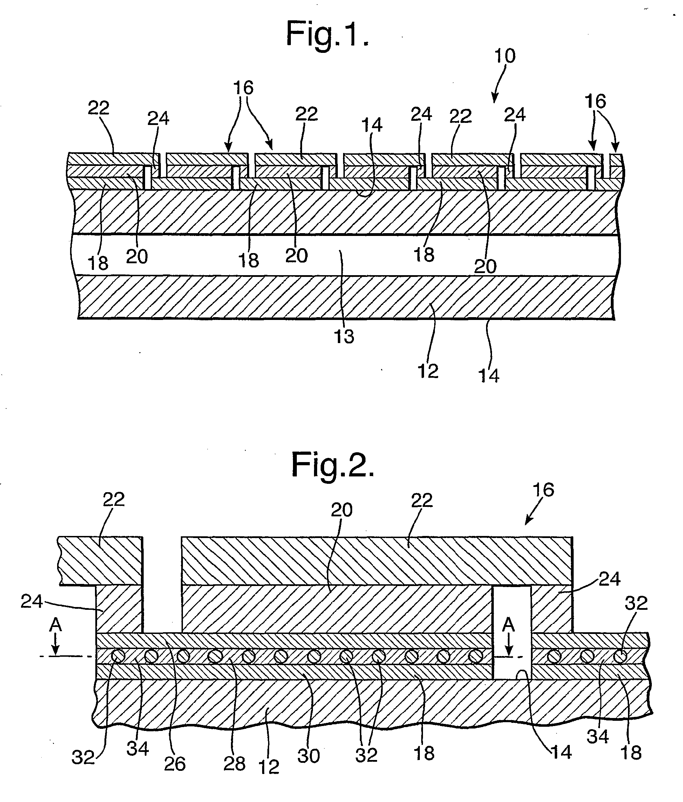

A solid oxide fuel cell module 10 according to the present invention is shown in figure 1 and comprises a hollow support member 12 and a plurality of solid oxide fuel cells 16 spaced apart longitudinally on at least one flat surface 14 of the hollow support member 12. The hollow support member 12 has one or more passages 13 extending longitudinally therethrough for the supply of one of the reactants, for example fuel e.g. hydrogen. Each solid oxide fuel cell 16 comprises an anode electrode 18, an electrolyte 20 and a cathode electrode 22. A plurality of interconnectors 24 are arranged to electrically connect the solid oxide fuel cells 16 in electrical series. Each interconnector 24 electrically connects an anode electrode 18 of one solid oxide fuel cell 16 to a cathode electrode 22 of an adjacent solid oxide fuel cell 16.

The anode electrode 18 of each solid oxide fuel cell 16 is arranged on the hollow support member 12, in the example where the fuel is supplied through the at least one passage 13 in the hollow support member 12. The electrolyte 20 of each solid oxide fuel cell 16 is arranged on the respective anode electrode 18 and the cathode electrode 22 of each solid oxide fuel cell 16 is arranged on the respective electrolyte 20.

The anode electrode 18 of each solid oxide fuel cell 16 comprises a first layer 26 adjacent the electrolyte 20, a second layer 28 on the first layer 26 and a third layer 30 on the second layer 28, as shown in figure 2. The first layer 26 comprises a nickel and ceramic cermet, the second layer 28 comprises a current collector 32 embedded in a nickel and ceramic cermet 34 and the third layer 30 comprises a nickel and ceramic cermet.

The current collector 32 may comprise nickel. The current collector 32 may comprise a nickel and ceramic cermet, the nickel and ceramic cermet of the current collector 32 having a higher concentration of nickel than the concentration of nickel in the nickel and ceramic cermet 34 of the first layer 26, second layer 28 and third layer 30. The current collector 32 may comprise a nickel and ceramic cermet, the nickel and ceramic cermet of the current collector 32 having different sized nickel and ceramic particles than the sizes of the nickel and ceramic particles of the nickel and ceramic cermet of the first layer 26, second layer 28 and third layer 30. The current collector 32 may comprise nickel and ceramic cermet, the nickel and ceramic cermet of the current collector 32 having less porosity than the nickel and ceramic cermet of the first layer 26, second layer 28 and third layer 30. The current collector 32 may comprise nickel and ceramic cermet, the nickel and ceramic cermet of the current collector 32 having different shaped and/or sized nickel and ceramic particles than the shapes and/or sizes of the nickel and ceramic particles of the nickel and ceramic cermet of the first layer 26, second layer 28 and third layer 30. The current collector 32 may comprise nickel and ceramic cermet, the nickel and ceramic cermet of the current collector 32 containing less pore formers than the nickel and ceramic cermet of the first layer 26, second layer 28 and third layer 30.

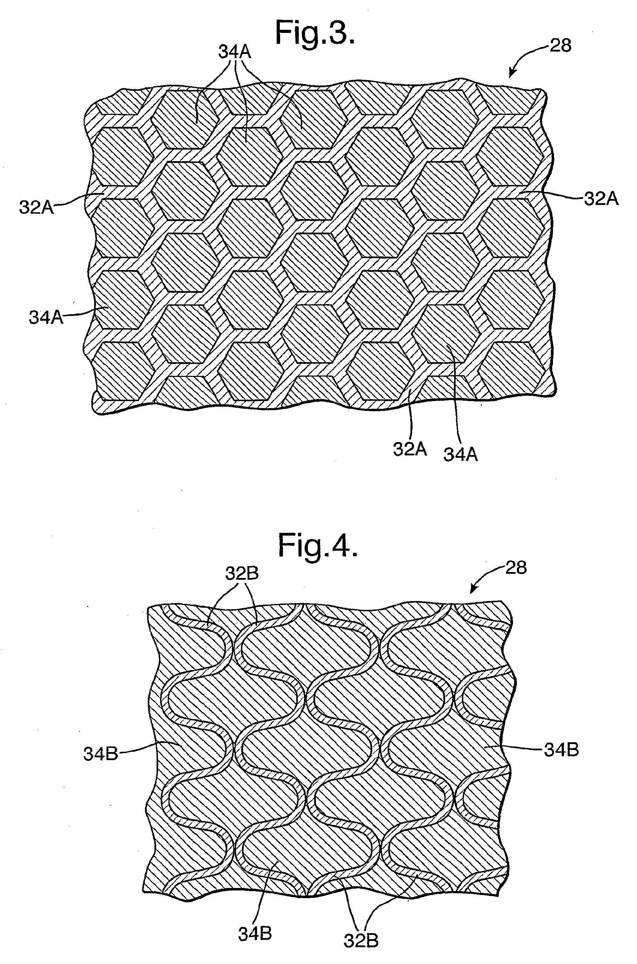

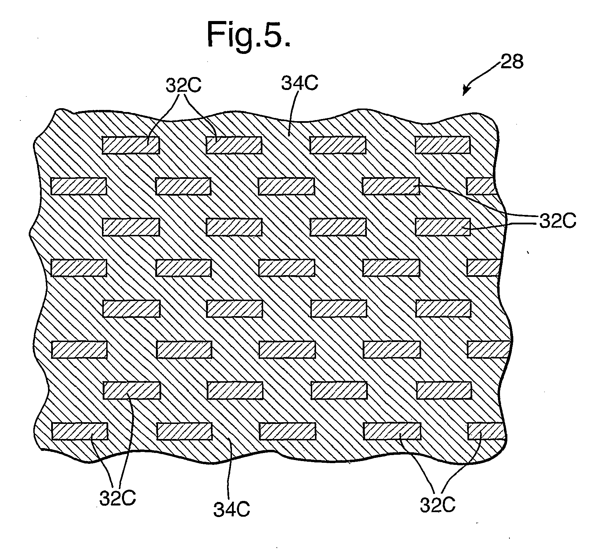

The second layer 28 may comprise a current collector mesh 32 embedded in a nickel and ceramic cermet 34 such that the length of the current collector mesh 32 is not continuous in any one direction. The second layer 28 preferably comprises a hexagonal current collector mesh 32A in a nickel and ceramic cermet 34A as shown in figure 3. Alternatively the second layer 28 comprises a sinusoidal current collector mesh 32B in a nickel and ceramic cermet 34B, as shown in figure 4. Alternatively the second layer 28 comprises a plurality of discontinuous current collector members 32C in a nickel and ceramic cermet 34C, as shown in figure 5. In the case of the current collector meshes 32A and 32B and the discontinuous current collector members 32C the arrangement is such as to minimise stresses in the anode electrode 18, while maximising in plane electrical conduction.

The layers of the solid oxide fuel cells are preferably deposited by screen-printing, inkjet printing or transfer printing directly on the other layers. Alternatively, the layers of the solid oxide fuel cells may be deposited by radio frequency sputtering, thermal spraying or plasma spraying, but with the use of masks to limit the deposition to the areas required on the other layers.

The nickel and ceramic cermet comprises nickel and yttria stabilised zirconia (YSZ) cermet or alternatively the nickel and ceramic cermet comprises nickel and ceria cermet or nickel and perovskite cermet.

The present invention provides a composite anode electrode with tailored microstructure to enhance in plane electrical conductance. The enhanced in plane electrical conductance is achieved by providing a current collector, (e.g. connected regions of the composite anode electrode with enhanced current collection capability) having more nickel and/or having less porosity, encapsulated in regions optimised for electrochemical reactions, having lots of reaction sites and a porous structure.

The present invention allows materials to be used in the current collector of the composite anode electrode, which have a coefficient of thermal expansion (CTE) that is mismatched with respect to that of the electrolyte or which generate a volume change during oxidation or reduction. In addition, the re-oxidation cycling stability of the composite anode electrode is enhanced. This is achieved by arranging the current collector in patterns/shapes such that it is not continuous in any one direction, hence reducing stresses.

The present invention allows materials to be used in the current collector of the composite anode electrode, which do not adhere/bond well to other solid oxide fuel cell materials. This is achieved by encapsulating the current collector within the anode electrode, so that only the anode electrode is in direct contact with the current collector and the adjacent layers and materials of the solid oxide fuel cells.

Additionally the present invention provides a composite anode electrode microstructure, which has an enhanced thermal stability. Over time at high temperatures small nickel particles tend to coarsen, which alters the microstructure of the anode electrode so that there are less percolations of the nickel particles and hence this leads to a decrease in the in plane electrical conductivity in a conventional anode electrode. The current collector of the composite anode electrode has a denser nickel microstructure than a conventional anode electrode and hence the nickel particles will coarsen at a slower rate than the small nickel particles in the anode electrode and this leads to a slower decrease in the in plane electrical conductivity of the whole composite anode electrode over time.

More broadly speaking, the first, second and third layers of the anode electrode may comprise other suitable metal and ceramic cermets, for example copper, palladium, platinum, rhenium or rhodium combined with yttria stabilised zirconia (YSZ), ceria or perovskite. Alternatively, the first, second and third layers of the anode electrode may comprise other suitable metal and ceramic cermets for example mixtures of two or more of copper, nickel, palladium, platinum, rhenium or rhodium combined with yttria stabilised zirconia (YSZ), ceria or perovskite.

The current collector may comprise other suitable metals, for example copper, palladium, platinum, rhenium or rhodium or alloys of copper, nickel, palladium, platinum, rhenium and rhodium. The current collector may comprise other metal and ceramic cermets for example copper, palladium, platinum, rhenium or rhodium combined with yttria stabilised zirconia (YSZ), ceria or perovskite again with a higher concentration of metal in the current collector than in the first, second and third layers of the anode electrode.

The current collector may comprise other suitable metals, for example copper, palladium, platinum, rhenium or rhodium or alloys of copper, nickel, palladium, platinum, rhenium and rhodium. The current collector may comprise other metal and ceramic cermets for example copper, palladium, platinum, rhenium or rhodium combined with yttria stabilised zirconia (YSZ), ceria or perovskite again with different sized metal and ceramic particles in the metal and ceramic of the current collector than the sizes of the metal and ceramic particles in the first, second and third layers of the anode electrode.

The current collector may comprise other suitable metals, for example copper, palladium, platinum, rhenium or rhodium or alloys of copper, nickel, palladium, platinum, rhenium and rhodium. The current collector may comprise other metal and ceramic cermets for example copper, palladium, platinum, rhenium or rhodium combined with yttria stabilised zirconia (YSZ), ceria or perovskite again with less porosity in the metal and ceramic of the current collector than in the metal and ceramic of the first, second and third layers of the anode electrode. The metal and ceramic particles of the current collector may again have different sized and/or shaped particles metal and ceramic particles or less pore formers than the metal and ceramic particles of the first, second and third layers.

Although the present invention has been described with reference to an anode electrode with a single current collector in the second layer of a three-layer anode electrode, it is equally possible to provide an anode electrode with other suitable numbers of current collectors and layers. For example two current collectors in the second and fourth layers of a five- layer anode electrode or three current collectors in the second, fourth and sixth layers of a seven-layer anode electrode, etc. However, it is preferred to use a single current collector in a three-layer anode electrode or two current collectors in a five-layer anode electrode to minimise material expense, manufacturing expense and complexity.

It is possible to arrange for the current collector to have a variation in the diameter, or cross-sectional area, of the holes in the hexagonal mesh of the current collector such that conduction perpendicular to the layers is different at different positions, for example to vary the conduction from one side to the other side of the anode electrode. It is possible to arrange for the current collector to have a variation in the diameter, or cross-sectional area, of the holes in the sinusoidal mesh of the current collector such that conduction perpendicular to the layers is different at different positions, for example to vary the conduction from one side to the other side of the anode electrode.

## Claims

1. A solid oxide fuel cell module (10) comprising a plurality of solid oxide fuel cells (16), each fuel cell (16) comprising an anode electrode (18), an electrolyte (20) and a cathode electrode (22), a plurality of interconnectors (24) being arranged to electrically connect the fuel cells (16) in electrical series, each interconnector (24) electrically connecting an anode electrode (18) of one fuel cell (16) to a cathode electrode (22) of an adjacent fuel cell (16), the anode electrode (18) comprising at least three layers (26, 28, 30), a first layer (26) being arranged on the electrolyte (20), a second layer (28) being arranged on the first layer (26) and a third layer (30) being arranged on the second layer (28), the first layer (26) comprising a metal and ceramic cermet, the third layer (30) comprising a metal and ceramic cermet, **characterised in that** the second layer (28) comprising a current collector (32) and a metal and ceramic cermet (34), the current collector (32) being embedded in the metal and ceramic cermet (34) of the second layer (28), the current collector (32) comprising a metal and ceramic cermet; the metal and ceramic cermet of the current collector (32) having a higher concentration of metal than the concentration of metal in the metal and ceramic cermet of the first layer (26), second layer (28) and third layer (30) or the metal and ceramic cermet of the current collector (32) having different sized metal and ceramic particles than the sizes of the metal and ceramic particles of the metal and ceramic cermet of the first layer (26), second layer (28) and third layer (30) or the metal and ceramic cermet of the current collector (32) having less porosity than the metal and ceramic cermet of the first layer (26), second layer (28) and third layer (30).

2. A solid oxide fuel cell module as claimed in claim 1 wherein the metal of the cermet comprising at least one of copper, nickel, palladium, platinum, rhenium or rhodium.

3. A solid oxide fuel cell module as claimed in claim 1 or claim 2 wherein the ceramic of the cermet comprising at least one of yttria stabilised zirconia, ceria or perovskite.

4. A solid oxide fuel cell module as claimed in claim 1 wherein the metal and ceramic cermet of the current collector (32) having different shaped and/or sized metal and ceramic particles than the shapes and/or sizes of the metal and ceramic particles of the metal and ceramic cermet of the first layer (26), second layer (28) and third layer (30).

5. A solid oxide fuel cell module as claimed in claim 1 wherein the metal and ceramic cermet of the current collector (32) containing less pore formers than the metal and ceramic cermet of the first layer (26), second layer (28) and third layer (30).

6. A solid oxide fuel cell module as claimed in any of claims 1 to 3 wherein the first layer (26) comprising a nickel and ceramic cermet, the second layer (28) comprising a current collector (32) embedded in a nickel and ceramic cermet and the third layer (30) comprising a nickel and ceramic cermet.

7. A solid oxide fuel cell module as claimed in claim 6 wherein the current collector (32) comprising nickel and ceramic cermet, the nickel and ceramic cermet of the current collector (32) having a higher concentration of nickel than the concentration of nickel in the nickel and ceramic cermet of the first layer (26), second layer (28) and third layer (30).

8. A solid oxide fuel cell module as claimed in claim 6 wherein the current collector (32) comprising nickel and ceramic cermet, the nickel and ceramic cermet of the current collector (32) having different sized nickel and ceramic particles than the sizes of the nickel and ceramic particles of the nickel and ceramic cermet of the first layer (26), second layer (28) and third layer (30).

9. A solid oxide fuel cell module as claimed in claim 6 wherein the current collector (32) comprising nickel and ceramic cermet, the nickel and ceramic cermet of the current collector (32) having less porosity than the nickel and ceramic cermet of the first layer (26), second layer (28) and third layer (30).

10. A solid oxide fuel cell module as claimed in claim 9 wherein the current collector (32) comprising nickel and ceramic cermet, the nickel and ceramic cermet of the current collector (32) having different shaped and/or sized nickel and ceramic particles than the shapes and/or sizes of the nickel and ceramic particles of the nickel and ceramic cermet of the first layer (26), second layer (28) and third layer (30).

11. A solid oxide fuel cell module as claimed in claim 9 wherein the current collector (32) comprising nickel and ceramic cermet, the nickel and ceramic cermet of the current collector (32) containing less pore formers than the nickel and ceramic cermet of the first layer (26), second layer (28) and third layer (30).

12. A solid oxide fuel cell module as claimed in any of claims 1 to 11 wherein the second layer (28) comprising a current collector mesh (32A, 32B) embedded in a metal and ceramic cermet.

13. A solid oxide fuel cell module as claimed in claim 12 wherein the second layer (28) comprising a hexagonal current collector mesh (32A) in a metal and ceramic cermet.

14. A solid oxide fuel cell module as claimed in claim 12 wherein the second layer (28) comprising a sinusoidal current collector mesh (32B) in a metal and ceramic cermet.

15. A solid oxide fuel cell module as claimed in any of claims 1 to 11 wherein the second layer (28) comprising a plurality of discontinuous current collector members (32C) in a metal and ceramic cermet.

**Patentansprüche**

1. Festoxid-Brennstoffzellenmodul (10), das eine Mehrzahl von Festoxid-Brennstoffzellen (16) umfasst, wobei jede Brennstoffzelle (16) eine Anodenelektrode (18), einen Elektrolyt (20) und eine Kathodenelektrode (22) umfasst, wobei eine Mehrzahl von Zwischenverbindungsgliedem (24) so angeordnet ist, dass sie die Brennstoffzellen (16) in elektrischer Reihe elektrisch verbinden, wobei jedes Zwischenverbindungsglied (24) eine Anodenelektrode (18) einer Brennstoffzelle (16) mit einer Kathodenelektrode (22) einer benachbarten Brennstoffzelle (16) elektrisch verbindet, wobei die Anodenelektrode (18) mindestens drei Schichten (26, 28, 30) umfasst, wobei eine erste Schicht (26) auf dem Elektrolyt (20) angeordnet ist, wobei eine zweite Schicht (28) auf der ersten Schicht (26) angeordnet ist, und wobei eine dritte Schicht (30) auf der zweiten Schicht (28) angeordnet ist, wobei die erste Schicht (26) einen Keramik-Metall-Verbundwerkstoff, Cermet, umfasst, wobei die dritte Schicht (30) einen Keramik-Metall-Verbundwerkstoff, Cermet, umfasst, **dadurch gekennzeichnet, dass** die zweite Schicht (28) einen Stromabnehmer (32) und einen Keramik-Metall-Verbundwerkstoff, Cermet, (34) umfasst, wobei der Stromabnehmer (32) in den Keramik-Metall-Verbundwerkstoff, Cermet, (34) der zweiten Schicht eingebettet ist, wobei der Stromabnehmer (32) einen Keramik-Metall-Verbundwerkstoff, Cermet, umfasst; wobei der Keramik-Metall-Verbundwerkstoff, Cermet, des Stromabnehmers (32) eine höhere Metallkonzentration als die Metallkonzentration in dem Keramik-Metall-Verbundwerkstoff, Cermet, der ersten Schicht (26), der zweiten Schicht (28) und der dritten Schicht (30) aufweist, oder wobei der Keramik-Metall-Verbundwerkstoff des Stromabnehmers (32) Keramik- und Metallpartikel anderer Größe als die Keramik- und Metallpartikel des Keramik-Metall-Verbundwerkstoffs, Cermet, der ersten Schicht (26), der zweiten Schicht (28) und der dritten Schicht (30) aufweist, oder wobei der Keramik-Metall-Verbundwerkstoff, Cermet, des Stromabnehmers (32) eine geringere Porosität aufweist als der Keramik-Metall-Verbundwerkstoff der ersten Schicht (26), der zweiten Schicht (28) und der dritten Schicht (30).

2. Festoxid-Brennstoffzellenmodul nach Anspruch 1, wobei das Metall des Keramik-Metall-Verbundwerkstoffs, Cermet, mindestens eines der folgenden Metalle umfasst: Kupfer, Nickel, Palladium, Platin, Rhenium oder Rhodium.

3. Festoxid-Brennstoffzellenmodul nach Anspruch 1 oder Anspruch 2, wobei der Keramikwerkstoff des Keramik-Metall-Verbundwerkstoffs, Cermet, mindestens eines der folgenden umfasst: Yttriumoxid, stabilisiertes Zirconiumoxid, Cerdioxid oder Perowskit.

4. Festoxid-Brennstoffzellenmodul nach Anspruch 1, wobei der Keramik-Metall-Verbundwerkstoff, Cermet, des Stromabnehmers (32) Keramik- und Metallpartikel in anderer Form und/oder anderer Größe als die Formen und/oder Größen der Keramik- und Metallpartikel des Keramik-Metall-Verbundwerkstoffs der ersten Schicht (26), der zweiten Schicht (28) und der dritten Schicht (30) aufweist.

5. Festoxid-Brennstoffzellenmodul nach Anspruch 1, wobei der Keramik-Metall-Verbundwerkstoff, Cermet, des Stromabnehmers (32) weniger Porenbilder aufweist als der Keramik-Metall-Verbundwerkstoff, Cermet, der ersten Schicht (26), der zweiten Schicht (28) und der dritten Schicht (30).

6. Festoxid-Brennstoffzellernnodul nach einem der Ansprüche 1 bis 3, wobei die erste Schicht (26) ein Nikke-Keramik-Cermet umfasst, wobei die zweite Schicht (28) einen Stromabnehmer (32) umfasst, der in ein Nicke-Keramik-Cermet eingebettet ist, und wobei die dritte Schicht (30) ein Nickel-Keramik-Cermet umfasst.

**7.** Festoxid-Brennstoffzellenmodul nach Anspruch 6, wobei der Stromabnehmer (32) ein Nickel-Keramik-Cermet umfasst, wobei das Nickel-Keramik-Cermet des Stromabnehmers (32) eine höhere Nickelkonzentration aufweist als die Nickelkonzentration in dem Nickel-Keramik-Cermet der ersten Schicht (26), der zweiten Schicht (28) und der dritten Schicht (30).

**8.** Festoxid-Brennstoffzellenmodul nach Anspruch 6, wobei der Stromabnehmer (32) ein Nickel-Keramik-Cermet umfasst, wobei das Nickel-Keramik-Cermet des Stromabnehmers (32) Nickel- und Keramikpartikel in anderen Größen als die Nickel- und Keramikpartikel des Nickel-Keramik-Cermet der ersten Schicht (26), der zweiten Schicht (28) und der dritten Schicht (30) aufweist.

**9.** Festoxid-Brennstoffzellenmodul nach Anspruch 6, wobei der Stromabnehmer (32) ein Nickel-Keramik-Cermet umfasst, wobei das Nickel-Keramik-Cermet des Stromabnehmers (32) eine geringere Porosität aufweist als die Porosität des Nickel-Keramik-Cermet der ersten Schicht (26), der zweiten Schicht (28) und der dritten Schicht (30).

**10.** Festoxid-Brennstoffzellenmodul nach Anspruch 9, wobei der Stromabnehmer (32) ein Nickel-Keramik-Cermet umfasst, wobei das Nickel-Keramik-Cermet des Stromabnehmers (32) Nickel- und Keramikpartikel in anderer Form und/oder in anderer Größe aufweist als die Nickel- und Keramikpartikel des Nickel-Keramik-Cermet der ersten Schicht (26), der zweiten Schicht (28) und der dritten Schicht (30).

**11.** Festoxid-Brennstoffzellenmodul nach Anspruch 9, wobei der Stromabnehmer (32) ein Nickel-Keramik-Cermet umfasst, wobei das Nickel-Keramik-Cermet des Stromabnehmers (32) weniger Porenformer aufweist als das Nickel-Keramik-Cermet der ersten Schicht (26), der zweiten Schicht (28) und der dritten Schicht (30).

**12.** Festoxid-Brennstoffzellenmodul nach einem der Ansprüche 1 bis 11, wobei der Stromabnehmer (32) ein Nickel-Keramik-Cermet umfasst, wobei die zweite Schicht (28) ein Stromabnehmernetz (32A, 32B) umfasst, das in einen Keramik-Metall-Verbundwerkstoff, Cermet, eingebettet ist.

**13.** Festoxid-Brennstoffzellenmodul nach Anspruch 12, wobei die zweite Schicht (28) ein hexagonales Stromabnehmernetz (32A) in einem Keramik-Metall-Verbundwerkstoff, Cermet, umfasst.

**14.** Festoxid-Brennstoffzellernnodul nach Anspruch 12, wobei die zweite Schicht (28) ein sinuskurvenförmiges Stromabnehmernetz (32A) in einem Keramik-Metall-Verbundwerkstoff, Cermet, umfasst.

**15.** Feststoff-Brennstoffzellenmodul nach einem der Ansprüche 1 bis 11, wobei die zweite Schicht (28) eine Mehrzahl unterbrochener Stromabnehmerelemente (32C) in einem Keramik-Metall-Verbundwerkstoff, Cermet, umfasst.

**Revendications**

**1.** Module de piles à combustible à oxyde solide (10) comprenant une pluralité de piles à combustible à oxyde solide (16), chaque pile à combustible (16) comprenant une électrode d'anode (18), un électrolyte (20) et une électrode de cathode (22), une pluralité d'interconnecteurs (24) étant disposés pour connecter électriquement les piles à combustible (16) en série électrique, chaque interconnecteur (24) connectant électriquement une électrode d'anode (18) d'une pile à combustible (16) à une électrode de cathode (22) d'une pile à combustible (16) adjacente, l'électrode d'anode (18) comprenant au moins trois couches (26, 28, 30), une première couche (26) étant disposée sur l'électrolyte (20), une deuxième couche (28) étant disposée sur la première couche (26) et une troisième couche (30) étant disposée sur la deuxième couche (28), la première couche (26) comprenant un mélange métal céramique, la troisième couche (30) comprenant un mélange métal céramique, **caractérisé en ce que** la deuxième couche (28) comprend un collecteur de courant (32) et un mélange métal céramique (34), le collecteur de courant (32) étant intégré au mélange métal céramique (34) de la deuxième couche (28), le collecteur de courant (32) comprenant un mélange métal céramique, le mélange métal céramique du collecteur de courant (32) ayant une plus forte concentration de métal que la concentration de métal dans le mélange métal céramique des première couche (26), deuxième couche (28) et troisième couche (30) ou le mélange métal céramique du collecteur de courant (32) ayant des particules de métal et de céramique de taille différente de celle des particules de métal et de céramique du mélange métal céramique des première couche (26), deuxième couche (28) et troisième couche (30) ou le mélange métal céramique du collecteur de courant (32) ayant une porosité inférieure à celle du mélange métal céramique des première couche (26), deuxième couche (28) et troisième couche (30).

**2.** Module de pile à combustible à oxyde solide selon la revendication 1, dans lequel le métal du mélange comprend du cuivre, du nickel, du palladium, du platine, du rhénium et/ou du rhodium.

**3.** Module de pile à combustible à oxyde solide selon la revendication 1 ou 2, dans lequel la céramique du mélange comprend de la zircone stabilisée à l'yttria, de l'oxyde de cérium et/ou de la pérovskite.

4. Module de pile à combustible à oxyde solide selon la revendication 1, dans lequel le mélange métal céramique du collecteur de courant (32) a des particules de métal et de céramique de forme et/ou taille différentes des formes et/ou tailles des particules de métal et de céramique du mélange métal céramique des première couche (26), deuxième couche (28) et troisième couche (30).

5. Module de pile à combustible à oxyde solide selon la revendication 1, dans lequel le mélange métal céramique du collecteur de courant (32) contient moins d'agents porogènes que le mélange métal céramique des première couche (26), deuxième couche (28) et troisième couche (30).

6. Module de pile à combustible à oxyde solide selon l'une quelconque des revendications 1 à 3, dans lequel la première couche (26) comprend un mélange nickel céramique, la deuxième couche (28) comprend un collecteur de courant (32) intégré dans un mélange nickel céramique et la troisième couche (30) comprend un mélange nickel céramique.

7. Module de pile à combustible à oxyde solide selon la revendication 6, dans lequel le collecteur de courant (32) comprend un mélange nickel céramique, le mélange nickel céramique du collecteur de courant (32) ayant une concentration en nickel supérieure à la concentration en nickel du mélange nickel céramique des première couche (26), deuxième couche (28) et troisième couche (30).

8. Module de pile à combustible à oxyde solide selon la revendication 6, dans lequel le collecteur de courant (32) comprend un mélange nickel céramique, le mélange nickel céramique du collecteur de courant (32) ayant des particules de nickel et de céramique d'une taille différente de la taille des particules de nickel et de céramique du mélange nickel céramique des première couche (26), deuxième couche (28) et troisième couche (30).

9. Module de pile à combustible à oxyde solide selon la revendication 6, dans lequel le collecteur de courant (32) comprend un mélange nickel céramique, le mélange nickel céramique du collecteur de courant (32) ayant une porosité inférieure à celle du mélange nickel céramique des première couche (26), deuxième couche (28) et troisième couche (30).

10. Module de pile à combustible à oxyde solide selon la revendication 9, dans lequel le collecteur de courant (32) comprend un mélange nickel céramique, le mélange nickel céramique du collecteur de courant (32) ayant des particules de nickel et de céramique d'une forme et/ou d'une taille différentes des formes et/ou tailles des particules de nickel et de céramique du mélange nickel céramique des première couche (26), deuxième couche (28) et troisième couche (30).

11. Module de pile à combustible à oxyde solide selon la revendication 9, dans lequel le collecteur de courant (32) comprend un mélange nickel céramique, le mélange nickel céramique du collecteur de courant (32) contenant moins d'agent porogènes que le mélange nickel céramique des première couche (26), deuxième couche (28) et troisième couche (30).

12. Module de pile à combustible à oxyde solide selon l'une quelconque des revendications 1 à 11, dans lequel la deuxième couche (28) comprend une maille de collecteur de courant (32A, 32B) intégrée à un mélange métal céramique.

13. Module de pile à combustible à oxyde solide selon la revendication 12, dans lequel la deuxième couche (28) comprend une maille de collecteur de courant hexagonale (32A) dans un mélange métal céramique.

14. Module de pile à combustible à oxyde solide selon la revendication 12, dans lequel la deuxième couche (28) comprend une maille de collecteur de courant sinusoïdale (32B) dans un mélange métal céramique.

15. Module de pile à combustible à oxyde solide selon l'une quelconque des revendications 1 à 11, dans lequel la deuxième couche (28) comprend une pluralité d'éléments formant collecteur de courant discontinu (32C) dans un mélange métal céramique.

# Fig.1.

# Fig.2.

## Fig.3.

## Fig.4.

# Fig.5.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 07245107 A
- US 5273837 A

- WO 0229917 A